(19) Europäisches Patentamt
European Patent Office
Office européen des brevets

(11) Veröffentlichungsnummer: **0 459 243 A2**

(12) **EUROPÄISCHE PATENTANMELDUNG**

(21) Anmeldenummer: **91108010.9**

(22) Anmeldetag: **17.05.91**

(51) Int. Cl.[5]: **C07D 239/38**, C07D 239/34, C07D 251/20, A01N 43/54, A01N 43/66

(30) Priorität: **30.05.90 DE 4017339**
**21.11.90 DE 4037003**

(43) Veröffentlichungstag der Anmeldung:
**04.12.91 Patentblatt 91/49**

(84) Benannte Vertragsstaaten:
**BE CH DE DK ES FR GB IT LI NL**

(71) Anmelder: **BAYER AG**

**W-5090 Leverkusen 1 Bayerwerk(DE)**

(72) Erfinder: **Drewes, Mark Wilhelm, Dr.**
**Grünstrasse 30**
**W-4018 Langenfeld(DE)**
Erfinder: **Kirsten, Rolf, Dr.**
**Carl-Langhans-Strasse 27**
**W-4019 Monheim(DE)**
Erfinder: **Krämer, Wolfgang, Dr.**
**Rosenkranz 25**
**W-5093 Burscheid 2(DE)**
Erfinder: **Krüger, Bernd-Wieland, Dr.**
**Unterboschbach 19**
**W-5060 Bergisch Gladbach 2(DE)**
Erfinder: **Santel, Hans-Joachim, Dr.**
**Grünstrasse 9a**
**W-5090 Leverkusen(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**

(54) **Substituierte Azine.**

(57) Die Erfindung betrifft neue substituierte Azine der Formel (I)

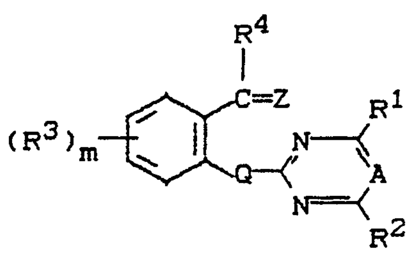

(I)

in welcher
m für die Zahlen 0, 1, 2 oder 3 steht,
A für Stickstoff oder eine C-X-Gruppierung steht, wobei X für Wasserstoff oder Halogen steht,
Q für Sauerstoff oder Schwefel steht,
Z für eine der nachstehenden Gruppierungen steht:
N-$R^5$ oder

EP 0 459 243 A2

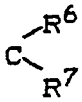 ,

und die Reste $R^1$ - $R^7$ die in der Beschreibung angegebene Bedeutung haben, ein Verfahren und neue Zwischenprodukte zu ihrer Herstellung und ihre Verwendung als Herbizide.

Die Erfindung betrifft neue substituierte Azine, ein Verfahren und neue Zwischenprodukte zu ihrer Herstellung sowie ihre Verwendung als Herbizide.

Eine Reihe von substituierten Azinen mit herbiziden Eigenschaften ist bereits bekannt (vergleiche JP-A 54117486 - Zit. in Chem. Abstracts 93, 150268c; EP-A 223 406; EP-A 249 708; JP-A 63258467 - Zit. in Chem. Abstracts 110, 130532a; JP-A 63258463 - Zit. in Chem. Abstracts 110, 192853q; EP-A 287 079; EP-A 374 839). Verbindungen aus den genannten Publikationen haben jedoch bisher keine größere Bedeutung erlangt.

Es wurden nun die neuen substituierten Azine der allgemeinen Formel (I) gefunden,

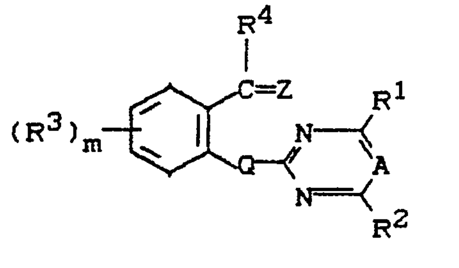

(I)

in welcher

| | |
|---|---|
| m | für die Zahlen 0, 1, 2 oder 3 steht, |
| A | für Stickstoff oder eine C-X-Gruppierung steht, wobei X für Wasserstoff oder Halogen steht, |
| Q | für Sauerstoff oder Schwefel steht, |
| $R^1$ und $R^2$ | gleich oder verschieden sind und für Wasserstoff, Halogen, Alkyl, Halogenalkyl, Alkoxyalkyl, Alkoxy, Halogenalkoxy, Alkylthio, Alkylamino oder Dialkylamino stehen, |
| $R^3$ | für Amino, Hydroxy, Halogen, Alkyl, Halogenalkyl, Alkoxy, Halogenalkoxy, Alkylamino, Dialkylamino, Alkylcarbonylamino, Alkoxycarbonylamino oder Alkylsulfonylamino steht, |
| $R^4$ | für Wasserstoff oder Alkyl steht und |
| Z | für eine der nachstehenden Gruppierungen steht: N-$R^5$ oder |

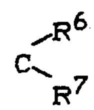

,

wobei

| | |
|---|---|
| $R^5$ | für Wasserstoff, Amino oder für jeweils gegebenenfalls substituiertes Alkyl, Alkenyl, Alkinyl, Aralkyl, Aryl, Alkoxycarbonyloxy, Arylaminocarbonyloxy, Carboxyalkoxy, Alkoxycarbonylalkoxy, Alkylamino, Dialkylamino, Aralkylamino, Arylamino, N-Alkyl-N-arylamino, Alkylcarbonylamino, Arylcarbonylamino, Heteroarylamino, Heteroarylcarbonylamino, Alkoxycarbonylamino, Alkylsulfonylamino oder Arylsulfonylamino steht, |
| $R^6$ | für Wasserstoff, Halogen, Cyano, Carboxy, Alkoxycarbonyl, Alkylcarbonylamino oder Dialkoxyphosphoryl steht und |
| $R^7$ | für Formyl, Cyano, Carboxy, Hydroxymethyl, Carbamoyl oder für jeweils gegebenenfalls substituiertes Alkoxycarbonyl, Cycloalkyloxycarbonyl, Aralkoxycarbonyl, Aryloxycarbonyl, Heterocyclylalkoxycarbonyl, Alkylthiocarbonyl, Aralkylthiocarbonyl, Arylthiocarbonyl, Alkylaminocarbonyl, Cycloalkylaminocarbonyl, Aralkylaminocarbonyl, Arylaminocarbonyl, Dialkylaminocarbonyl, Alkylhydrazinocarbonyl, Arylhydrazinocarbonyl, Pyrrolidinylcarbonyl, Piperidinylcarbonyl, Morpholinylcarbonyl, Piperazinylcarbonyl, Phthalimidoxycarbonyl, für die Gruppierung |

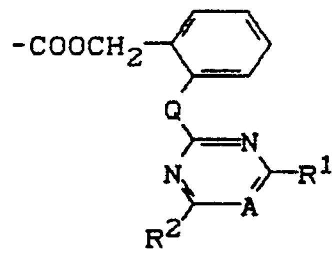

in welcher A, Q, $R^1$ und $R^2$ die oben angegebenen Bedeutungen haben, steht oder zusammen mit $R^6$ für die Gruppierung $-CO-O-(CH_2)_n-$ steht, wobei n für die Zahlen 1 bis 4 steht.

Man erhält die neuen substituierten Azine der allgemeinen Formel (I), wenn man entsprechende Carbonylverbindungen der allgemeinen Formel (II)

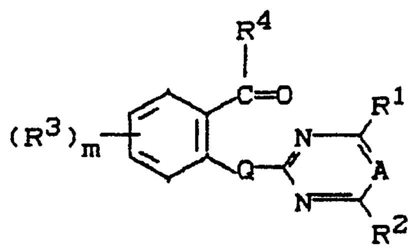

(II)

in welcher

m, A, Q, $R^1$, $R^2$, $R^3$ und $R^4$ die oben angegebene Bedeutung haben,

mit Amino- oder Methylen-Verbindungen der allgemeinen Formel (III)

$H_2Z$ (III)

in welcher

Z die oben angegebene Bedeutung hat,

gegebenenfalls in Gegenwart eines Reaktionshilfsmittels und gegebenenfalls in Gegenwart eines Verdünnungsmittels umsetzt und gegebenenfalls die hierbei erhaltenen Produkte anschließend nach üblichen Methoden in andere Derivate gemäß der Definition der Verbindungen der Formel (I) umwandelt.

Die neuen substituierten Azine der allgemeinen Formel (I) zeichnen sich durch starke Herbizidwirkung aus.

Die Erfindung betrifft vorzugsweise Verbindungen der Formel (I), in welcher

m für die Zahlen 0, 1 oder 2 steht,

A für Stickstoff oder eine C-X-Gruppierung steht, wobei X für Wasserstoff oder Halogen steht,

Q für Sauerstoff oder Schwefel steht,

$R^1$ und $R^2$ gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen,

$R^3$ für Amino, Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, Di-($C_1$-$C_2$-alkyl)-amino, $C_1$-$C_4$-Alkyl-carbonylamino, $C_1$-$C_4$-Alkoxy-carbonylamino oder $C_1$-$C_4$-Alkylsulfonylamino steht,

$R^4$ für Wasserstoff oder $C_1$-$C_4$-Alkyl steht und

Z für eine der nachstehenden Gruppierungen steht:

$N-R^5$ oder

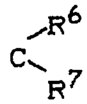 ,

wobei

$R^5$ für Wasserstoff, Amino oder für jeweils gegebenenfalls durch Halogen substituiertes $C_1$-$C_6$-Alkyl, $C_3$-$C_6$-Alkenyl, $C_3$-$C_6$-Alkinyl, Carboxy-$C_1$-$C_2$-alkoxy, $C_1$-$C_6$-Alkoxycarbonyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkyl-carbonylamino, $C_1$-$C_6$-Alkoxy-carbonylamino, $C_1$-$C_6$-Alkyl-sulfonylamino, oder für jeweils gegebenenfalls durch Nitro, Hydroxy, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenylaminocarbonyloxy, Phenylamino, Phenyl-$C_1$-$C_4$-alkylamino, N-($C_1$-$C_4$-alkyl)-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Halogen, Cyano, Carboxy, $C_1$-$C_6$-Alkoxy-carbonyl, $C_1$-$C_6$-Alkylcarbonylamino oder Di-($C_1$-$C_4$-alkoxy)-phosphoryl steht und

$R^7$ für Formyl, Cyano, Carboxy, Hydroxymethyl, Carbamoyl oder für jeweils gegebenenfalls durch Halogen, Carboxy oder $C_1$-$C_4$-Alkoxy-carbonyl substituiertes $C_1$-$C_6$-Alkoxy-carbonyl, $C_5$-$C_6$-Cycloalkyloxy-carbonyl, $C_1$-$C_6$-Alkylthio-carbonyl, $C_1$-$C_6$-Alkylamino-carbonyl oder $C_5$-$C_6$-Cycloalkylamino-carbonyl, für Di-($C_1$-$C_2$-alkyl)-amino-carbonyl, für $C_1$-$C_4$-Alkylamino-carbonyl-$C_1$-$C_4$-alkoxy-carbonyl, für Di-($C_1$-$C_2$-alkyl)-amino-carbonyl-$C_1$-$C_4$-alkoxy-carbonyl, für Phenylaminocarbonyl-$C_1$-$C_4$-alkoxy-carbonyl, für N-Methyl-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylamino-carbonyl, N-($C_1$-$C_4$-Alkyl)-phenylamino-carbonyl oder Phenylhydrazinocarbonyl, für Phthalimidoxycarbonyl, für die Gruppierung

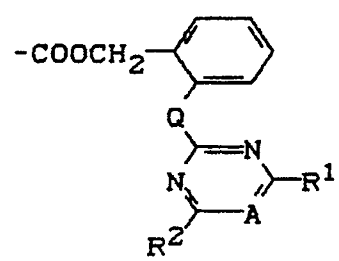

in welcher A, Q, $R^1$ und $R^2$ die oben als bevorzugt angegebenen Bedeutungen haben, steht oder zusammen mit $R^5$ für die Gruppierung $-CO-O-(CH_2)_n-$ steht, wobei n für die Zahlen 2 oder 3 steht.

Die in der Definition der erfindungsgemäßen Verbindungen aufgeführten aliphatischen Kohlenwasserstoffreste (z.B. Alkyl, Alkenyl, Alkinyl), auch in Kombination mit Heteroatomen (z.B. in Alkoxy, Alkylthio, Alkylamino), sind jeweils geradkettig oder verzweigt.

Halogen steht jeweils im allgemeinen für Fluor, Chlor, Brom oder Jod, vorzugsweise für Fluor, Chlor oder Brom, insbesondere für Fluor oder Chlor.

Die Erfindung betrifft insbesondere Verbindungen der Formel (I), in welcher

m für die Zahlen 0, 1 oder 2 steht,

A für Stickstoff oder eine CH-Gruppierung steht,

Q für Sauerstoff steht,

$R^1$ für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino steht,

$R^2$ für Wasserstoff, Chlor, Methyl, Ethyl, Trifluormethyl, Methoxy, Ethoxy, Difluormethoxy, Methylthio, Methylamino, Ethylamino oder Dimethylamino steht,

$R^3$ für Amino, Hydroxy, Fluor, Chlor, Brom, Methyl, Ethyl, Trifluormethyl, Methoxy, Difluormethoxy, Trifluormethoxy, Methylamino, Dimethylamino, Acetylamino, Methoxycarbonylamino oder Methylsulfonylamino steht,

$R^4$ für Wasserstoff oder Methyl steht und

Z für eine der nachstehenden Gruppierungen steht:
N-$R^5$ oder

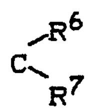,

wobei

$R^5$ für Wasserstoff, Amino, für Methyl, Ethyl, Propyl, Isopropyl, Butyl, Isobutyl, sec-Butyl, tert-Butyl, Allyl, Propargyl, Carboxymethoxy, Carboxyethoxy, Methoxycarbonyloxy, Ethoxycarbonyloxy, Propoxycarbonyloxy, Butoxycarbonyloxy, Methoxycarbonylmethoxy, Ethoxycarbonylmethoxy, Methoxycarbonylethoxy, Ethoxycarbonylethoxy, Methylamino, Ethylamino, Propylamino, Isopropylamino, Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Hydroxy, Cyano, Fluor, Chlor, Brom, Methyl, Trifluormethyl, Methoxy, Trifluormethoxy, Methylthio oder Trifluormethylthio substituiertes Phenyl, Benzyl, Phenylaminocarbonyloxy, Phenylamino, Benzylamino, N-Methyl-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Fluor, Chlor, Cyano, Carboxy, $C_1$-$C_4$-Alkoxy-carbonyl, $C_1$-$C_4$-Alkyl-carbonylamino, Dimethoxyphosphoryl oder Diethoxyphosphoryl steht und

$R^7$ für Formyl, Cyano, Carboxy, Hydroxymethyl, Carbamoyl, für jeweils gegebenenfalls durch Fluor, Chlor, Carboxy oder $C_1$-$C_4$-Alkoxy-carbonyl substituiertes $C_1$-$C_4$-Alkoxy-carbonyl, $C_5$-$C_6$-Cycloalkyloxy-carbonyl, $C_1$-$C_4$-Alkylthiocarbonyl, $C_1$-$C_4$-Alkylamino-carbonyl oder $C_5$-$C_6$-Cycloalkylamino-carbonyl, für Dimethylamino-carbonyl, für $C_1$-$C_4$-Alkylaminocarbonyl-$C_1$-$C_4$-alkoxy-carbonyl, für Dimethylaminocarbonyl-$C_1$-$C_4$-alkoxy-carbonyl, für N-Methyl-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinyl-carbonyl, Morpholinylcarbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-phenylaminocarbonyl, Phenylhydrazinocarbonyl, für Phthalimidoxycarbonyl, für die Gruppierung

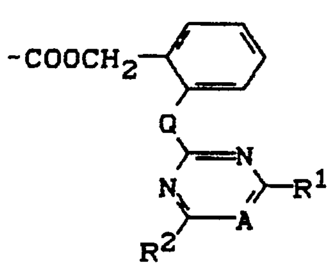

in welcher A, Q, $R^1$ und $R^2$ die oben als insbesondere bevorzugt angegebenen Bedeutungen haben, steht oder zusammen mit $R^6$ für die Gruppierung $-CO-O-CH_2CH_2-$ steht.

Ganz besonders bevorzugt werden die Verbindungen der Formel (I), in welcher

m für die Zahlen 0, 1 oder 2 steht,
A für eine CH-Gruppierung steht,
Q für Sauerstoff steht,
$R^1$ für Methoxy steht,
$R^2$ für Methoxy steht,
$R^3$ für Fluor, Chlor oder Methyl steht,
$R^4$ für Wasserstoff steht und
Z die oben als insbesondere bevorzugt angegebene Bedeutung hat.

Verwendet man für das erfindungsgemäße Verfahren zur Herstellung der Verbindungen der Formel (I) beispielsweise 2-(4,6-Dimethoxy-pyrimidin-2-yl-oxy)-benzaldehyd und Acethydrazid als Ausgangsstoffe, so kann der Reaktionsablauf durch das folgende Formelschema wiedergegeben werden:

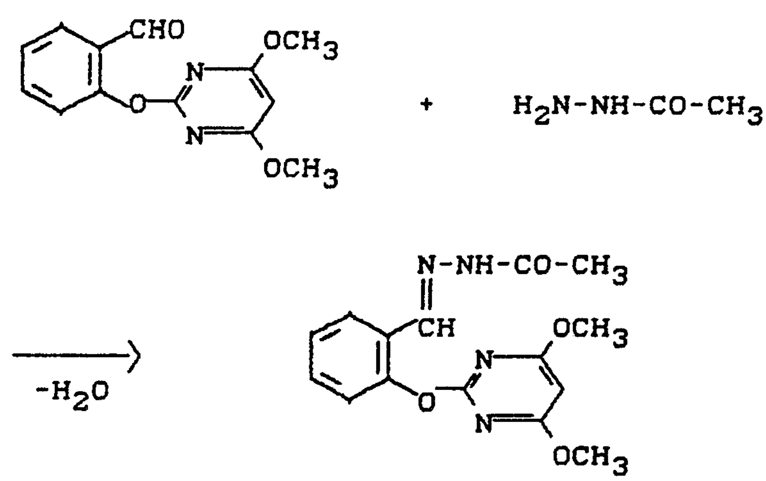


Die beim erfindungsgemäßen Verfahren zur Herstellung von Verbindungen der Formel (I) als Ausgangsstoffe zu verwendenden Carbonylverbindungen sind durch die Formel (II) allgemein definiert,

In Formel (II) haben A, Q, $R^1$, $R^2$, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, Q, $R^1$, $R^2$, $R^3$ und $R^4$ angegeben wurden.

Als Beispiele für die Ausgangsstoffe der Formel (II) seien genannt:
2-(4,6-Dimethyl-pyrimidin-2-yl-oxy)-,
2-(4-Methoxy-6-methyl-pyrimidin-2-yl-oxy)-,
2-(4,6-Dimethoxy-pyrimidin-2-yl-oxy)-,
2-(4-Methoxy-6-trifluormethyl-pyrimidin-2-yl-oxy)-,
2-(4,6-Dimethyl-s-triazin-2-yl-oxy)-,
2-(4-Methoxy-6-methyl-s-triazin-2-yl-oxy)- und
2-(4,6-Dimethoxy-s-triazin-2-yl-oxy)-benzaldehyd.

Die Ausgangsstoffe der Formel (II) sind bekannt und/oder können nach an sich bekannten Verfahren hergestellt werden (vergleiche EP-A 223 406; EP-A 249 708; JP-A 63258467 - Zit. in Chem. Abstracts 110, 130532a; JP-A 63258463 - Zit. in Chem. Abstracts 110, 192853q; EP-A 287 079).

Neu und Gegenstand der vorliegenden Anmeldung sind die Ausgangsverbindungen der allgemeinen Formel (IIa)

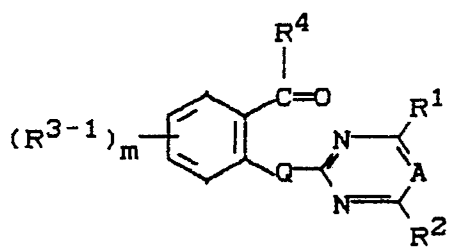

(IIa)

in welcher

A, Q, $R^1$, $R^2$ und $R^4$ die oben angegebene Bedeutung haben,
m für die Zahlen 1 oder 2 steht und
$R^{3-1}$ für Fluor und gegebenenfalls zusätzlich für Chlor steht.

In Formel (IIa) haben A, Q, $R^1$, $R^2$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, Q, $R^1$, $R^2$ und $R^4$ angegeben wurden; m steht vorzugsweise für die Zahlen 1 oder 2 und $R^{3-1}$ steht vorzugsweise für Fluor.

Man erhält die neuen Verbindungen der Formel (IIa), wenn man Halogenverbindungen der allgemeinen Formel (IV)

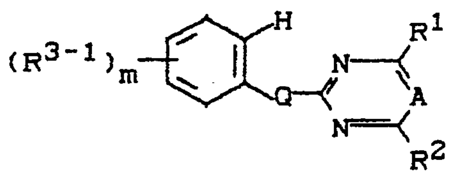
(IV)

in welcher

m, A, Q, $R^1$, $R^2$ und $R^{3-1}$ die oben bei Formel (IIa) angegebene Bedeutung haben,

mit einem Metallierungsmittel, wie z.B. Butyllithium, in Gegenwart eines inerten Verdünnungsmittels, wie z.B. Tetrahydrofuran, bei Temperaturen zwischen -20° C und -120° C, vorzugsweise zwischen -50° C und -80° C behandelt, anschließend in situ mit Carbonsäureamiden der allgemeinen Formel (V)

$R^4$-CO-$NR_2$ (V)

in welcher

$R^4$ die oben angegebene Bedeutung hat und
R für Alkyl steht,

bei Temperaturen zwischen 0° c und -80° C umsetzt und nach üblichen Methoden aufarbeitet (vgl. die Herstellungsbeispiele).

In Formel (IV) haben A, Q, $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, Q, $R^1$ und $R^2$ angegeben wurden; m steht vorzugsweise für die Zahlen 1 oder 2 und $R^{3-1}$ steht vorzugsweise für Fluor.

Die Halogenverbindungen der Formel (IV) sind noch nicht aus der Literatur bekannt und sind gleichfalls Gegenstand der vorliegenden Anmeldung.

Man erhält die neuen Verbindungen der Formel (IV), wenn man halogenierte (Thio)Phenole der Formel (VI)

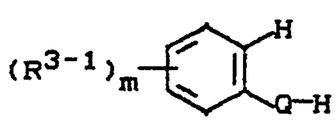
(VI)

in welcher

m, Q und $R^{3-1}$ die oben angegebene Bedeutung haben,

mit reaktionsfähigen Azinen der Formel (VII)

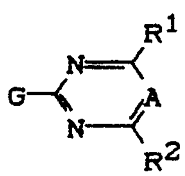
(VII)

in welcher

A, $R^1$ und $R^2$ die oben angegebene Bedeutung haben und

G für eine nucleofuge Abgangsgruppe steht,

gegebenenfalls in Gegenwart eines Säureakzeptors, wie Kaliumcarbonat, und gegebenenfalls in Gegenwart eines Verdünnungsmittels, wie z.B. Acetonitril, bei Temperaturen zwischen 20 °C und 120 °C umsetzt (vgl. die Herstellungsbeispiele).

In Formel (VI) stehen vorzugsweise m für die Zahlen 1 oder 2, Q für Sauerstoff und $R^{3-1}$ für Fluor.

Die halogenierten (Thio)Phenole der Formel (VI) sind bekannte Synthesechemikalien.

In Formel (VII) haben A, $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^1$ und $R^2$ angegeben wurden; G steht vorzugsweise für Chlor oder Methylsulfonyl.

Die reaktionsfähigen Azine der Formel (VII) sind bekannt und/oder können nach an sich bekannten Verfahren hergestellt werden (vgl. J. Chem. Soc. 1957, 1830, 1833; J. Org. Chem. 26 (1961), 792; US-P 3308119; US-P 4711959).

In Formel (V) hat $R^4$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^4$ angegeben wurde; R steht vorzugsweise für $C_1$-$C_4$-Alkyl, insbesondere für Methyl.

Die Carbonsäureamide der Formel (V) sind bekannte Synthesechemikalien.

Die beim erfindungsgemäßen Verfahren zur Herstellung von Verbindungen der Formel (I) weiter als Ausgangsstoffe zu verwendenden Amino- oder Methylen-Verbindungen sind durch die Formel (III) allgemein definiert.

In Formel (III) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Z angegeben wurde.

Als Beispiele für die Ausgangsstoffe der Formel (III) seien genannt:

Ammoniak, Hydroxylamin, Hydrazin, Methylamin, Ethylamin, Propylamin, Isopropylamin, Butylamin, Isobutylamin, sec-Butylamin, tert-Butylamin, Allylamin, Propargylamin, O-Methyl-, O-Ethyl-, O-Propyl-, O-Isopropyl-, O-Butyl-, O-Isobutyl- und O-sec-Butyl-hydroxylamin, O-Allyl-hydroxylamin, Aminooxyessigsäure-methylester und -ethylester, α-Aminooxy-propionsäure-methylester und -ethylester, Methylhydrazin, Ethylhydrazin, Propylhydrazin, Isopropylhydrazin, Butylhydrazin, Isobutylhydrazin, sec-Butyl-hydrazin, tert-Butylhydrazin, N,N-Dimethylhydrazin, Acethydrazid, Propionylhydrazid, Methoxycarbonylhydrazin, Ethoxycarbonylhydrazin, Methylsulfonylhydrazin, Ethylsulfonylhydrazin, Phenylhydrazin, Benzoylhydrazin, Benzolsulfonsäurehydrazid, p-Toluolsulfonsäurehydrazid, Malonsäure, Cyanessigsäure, Malonsäuredinitril, Cyanessigsäure-methylester und -ethylester, Malonsäure-dimethylester und -diethylester, γ-Butyrolacton.

Die Ausgangsstoffe der Formel (III) sind bekannte Synthesechemikalien.

Das erfindungsgemäße Verfahren zur Herstellung der neuen substituierten Azine der Formel (I) wird vorzugsweise unter Verwendung von Verdünnungsmitteln durchgeführt. Als Verdünnungsmittel kommen dabei neben Wasser praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren wird gegebenenfalls in Gegenwart eines Reaktionshilfsmittels durchgeführt. Geeignete Reaktionshilfsmittel sind Stoffe, die üblicherweise zur Steuerung und/oder Beschleunigung von Kondensationsreaktionen zwischen Carbonylverbindungen und Amino- oder Methylen-Verbindungen verwendet werden. Hierzu gehören insbesondere Stickstoffverbindungen, wie z.B. Ammoniumacetat, β-Alanin, Pyridin und Piperidin.

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 120 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im

allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Reaktionshilfmittels durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vergleiche die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum, Mercurialis.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfen-anlagen, auf Zier- und Sportrasen und Weideflächen und zur selektiven Unkrautbekämpfung in einjährigen Kulturen eingesetzt werden.

Die erfindungsgemäßen Verbindungen der Formel (I) können sowohl im Vorauflauf- als auch im Nachauflauf-Verfahren zur Bekämpfung von monokotylen und dikotylen Unkräutern eingesetzt werden.

In gewissem Umfang zeigen die erfindungsgemäßen Verbindungen auch fungizide Wirkung, z.B. gegen Pyricularia oryzae.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); Ethyl-2-{[(4-chlor-6-methoxy-2-pyrimidinyl)-aminocarbonyl]-aminosulfonyl}-benzoat (CHLORIMURON); N,N-Dimethyl-N'-(3-chlor-4-methylphenyl)-harnstoff (CHLORTOLURON); 2-Chlor-4-ethylamino-6-(3-cyanopropylamino)-1,3,5-triazin (CYANAZIN); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [-(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); N-Phosphonomethylglycin (GLYPHOSATE); 2-{4-[(3-Chlor-5-(trifluormethyl)-2-pyridinyl)-oxy]-phenoxy}-propansäure bzw. deren Ethylester (HALOXYFOP); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 2-[1-(Ethoxamino)-butyliden]-5-(2-ethylthiopropyl)-1,3-cyclohexadion (SETHOXYDIM); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) und 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01

und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

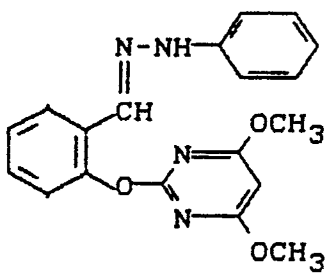

Eine Mischung aus 2,7 g (10 mMol) 2-(4,6-Dimethoxy-pyrimidin-2-yl-oxy)-benzaldehyd, 1,1 g (10 mMol) Phenylhydrazin und 40 ml Toluol wird 2 Stunden bei 20 °C gerührt und anschließend eingeengt. Der Rückstand wird mit Ethanol / Wasser (1:1) zur Kristallisation gebracht und das kristalline Produkt durch Absaugen isoliert.

Man erhält 2,6 g (75 % der Theorie) 2-(4,6-Dimethoxy-pyrimidin-2-yl-oxy)-benzaldehyd-phenylhydrazon vom Schmelzpunkt 112 °C.

Beispiel 2

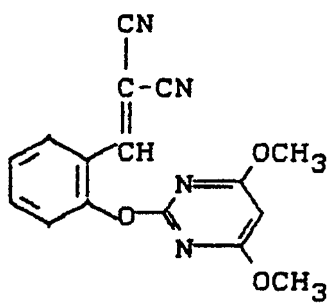

Eine Mischung aus 2,6 g (10 mMol) 2-(4,6-Dimethoxy-pyrimidin-2-yl-oxy)-benzaldehyd, 0,67 g (10 mMol) Malonsäuredinitril, 0,1 g Ammoniumacetat und 80 ml Toluol wird 5 Stunden am Wasserabscheider zum Sieden erhitzt. Anschließend wird eingeengt und der Rückstand mit Diethylether/Petrolether zur Kristallisation gebracht. Das kristalline Produkt wird durch Absaugen isoliert.

Man erhält 2,2 g (71 % der Theorie) α-(2-(4,6-Dimethoxypyrimidin-2-yl-oxy)-phenyl)-methylen-malonsäuredinitril vom Schmelzpunkt 123 °C.

Analog zu den Beispielen 1 oder 2 sowie entsprechend der allgemeinen Beschreibung des erfindungsgemäßen Herstellungsverfahrens oder durch Folgereaktionen nach üblichen Methoden können beispielsweise auch die in der nachstehenden Tabelle 1 aufgeführten Verbindungen der Formel (I) hergestellt werden.

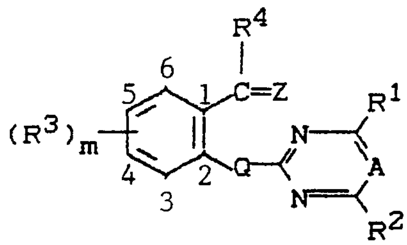

(I)

(Q ist stets in 2-Position des Benzolringes gebunden.)

<u>Tabelle 1:</u> Beispiele für die Verbindungen der Formel (I)

| Beisp.-Nr. | A | Q | $R^1$ | $R^2$ | $R^3$ | $R^4$ | m | Z | Schmelz-punkt (°C) |
|---|---|---|---|---|---|---|---|---|---|
| 3 | CH | O | $OCH_3$ | $OCH_3$ | - | H | 0 | $CHCOOC_2H_5$ | 70 |
| 4 | N | O | $OCH_3$ | $OCH_3$ | - | H | 0 | CHCOOH | 196 |
| 5 | N | O | $OCH_3$ | $OCH_3$ | - | H | 0 | $CHCOOC_2H_5$ | 128 |
| 6 | CH | O | $OCH_3$ | $OCH_3$ | - | H | 0 | $CHCH_2OH$ | (amorph) |
| 7 | CH | O | $OCH_3$ | $OCH_3$ | - | H | 0 | 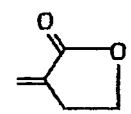 | 124 |
| 8 | CH | O | $OCH_3$ | $OCH_3$ | - | H | 0 | 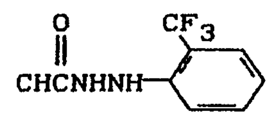 | 147 |
| 9 | CH | O | $OCH_3$ | $OCH_3$ | (5-)F | H | 1 | 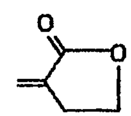 | 126 |
| 10 | N | O | $OCH_3$ | $OCH_3$ | (5-)F | H | 1 | 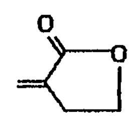 | 139 |

Tabelle 1: Beispiele für die Verbindungen der Formel (I) (Fortsetzung)

| Beisp.-Nr. | A | Q | $R^1$ | $R^2$ | $R^3$ | $R^4$ | m | Z | Schmelzpunkt (°C) |
|---|---|---|---|---|---|---|---|---|---|
| 11 | CH | O | $OCH_3$ | $OCH_3$ | - | H | 0 | $C(COOC_2H_5)_2$ | (amorph) |
| 12 | CH | O | $OCH_3$ | $OCH_3$ | (5-)F | H | 1 | CHCOOH | 193 |
| 13 | N | O | $OCH_3$ | $OCH_3$ | - | H | 0 | $CHCH_2OH$ | (amorph) |
| 14 | CH | O | $OCH_3$ | $OCH_3$ | - | H | 0 | $CHCOOCH(CH_3)_2$ | (amorph) |
| 15 | CH | O | $OCH_3$ | $OCH_3$ | (5-)$CF_3$ | H | 1 | CHCOOH | (amorph) |
| 16 | CH | O | $OCH_3$ | $OCH_3$ | - | H | 0 | 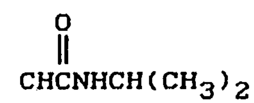 | 131 |
| 17 | CH | O | $OCH_3$ | $OCH_3$ | - | H | 0 | 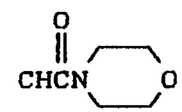 | 182 |
| 18 | CH | O | $OCH_3$ | $OCH_3$ | - | H | 0 | 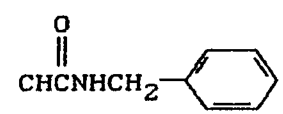 | 153 |
| 19 | CH | O | $OCH_3$ | $OCH_3$ | - | H | 0 | 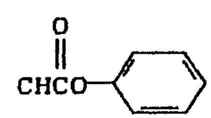 | (amorph) |

Tabelle 1: Beispiele für die Verbindungen der Formel (I) (Fortsetzung)

| Beisp.-Nr. | A | Q | $R^1$ | $R^2$ | $R^3$ | $R^4$ | m | Z | Schmelzpunkt (°C) |
|---|---|---|---|---|---|---|---|---|---|
| 20 | CH | O | $OCH_3$ | $OCH_3$ | - | H | 0 | 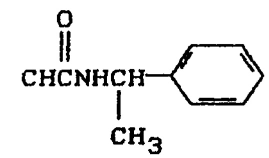 | (amorph) |
| 21 | CH | O | $OCH_3$ | $OCH_3$ | - | H | 0 | $CHC(=O)O(CH_2)_3CH_3$ | (amorph) |
| 22 | CH | O | $OCH_3$ | $OCH_3$ | (4-)F | H | 1 | CHCOOH | 179 |
| 23 | CH | O | $OCH_3$ | $OCH_3$ | (5-)F | H | 1 | $CHCH_2OH$ | (amorph) |
| 24 | CH | O | $OCH_3$ | $OCH_3$ | (4-)F | H | 1 | $CHCH_2OH$ | (amorph) |
| 25 | CH | O | $OCH_3$ | $OCH_3$ | (4-)F | H | 1 | $C(COOC_2H_5)_2$ | (amorph) |
| 26 | CH | O | $OCH_3$ | $OCH_3$ | - | H | 0 | CHCHO | 94 |
| 27 | CH | O | $OCH_3$ | $OCH_3$ | (5-)$CH_3$ | H | 1 | CHCOOH | 162 |
| 28 | CH | O | $OCH_3$ | $OCH_3$ | (5-)Cl | H | 1 | CHCOOH | 200 |

Tabelle 1: Beispiele für die Verbindungen der Formel (I) (Fortsetzung)

| Beisp.-Nr. | A | Q | $R^1$ | $R^2$ | $R^3$ | $R^4$ | m | Z | Schmelzpunkt (°C) |
|---|---|---|---|---|---|---|---|---|---|
| 29 | CH | O | $OCH_3$ | $OCH_3$ | (5-)Cl | H | 1 | $C(COOC_2H_5)_2$ | (amorph) |
| 30 | CH | O | $OCH_3$ | $OCH_3$ | (5-)Cl | H | 1 | $CHCH_2OH$ | (amorph) |
| 31 | CH | O | $OCH_3$ | $OCH_3$ | (5-)$CH_3$ | H | 1 | $CHCH_2OH$ | (amorph) |
| 32 | CH | O | $CH_3$ | $OCH_3$ | - | H | 0 | $C(COOC_2H_5)_2$ | (amorph) |
| 33 | CH | O | $OCH_3$ | $OCH_3$ | - | H | 0 | 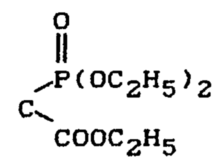 | (amorph) |
| 34 | CH | O | $OCH_3$ | $OCH_3$ | - | H | 0 | 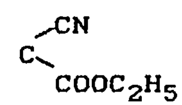 | 123 |
| 35 | CH | O | $OCH_3$ | $OCH_3$ | - | H | 0 | 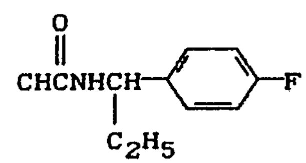 | (amorph) |
| 36 | CH | O | $OCH_3$ | $OCH_3$ | - | H | 0 | 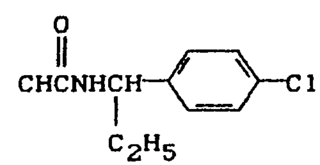 | (amorph) |

Tabelle 1: Beispiele für die Verbindungen der Formel (I) (Fortsetzung)

| Beisp.-Nr. | A | Q | $R^1$ | $R^2$ | $R^3$ | $R^4$ | m | Z | Schmelzpunkt (°C) |
|---|---|---|---|---|---|---|---|---|---|
| 37 | CH | O | $OCH_3$ | $OCH_3$ | - | H | 0 | 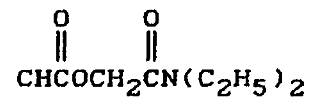 | (amorph) |
| 38 | CH | O | $OCH_3$ | $OCH_3$ | - | H | 0 | 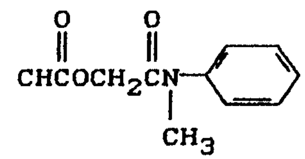 | (amorph) |
| 39 | CH | O | $OCH_3$ | $OCH_3$ | - | H | 0 | CHCN | (amorph) |
| 40 | CH | O | $OCH_3$ | $OCH_3$ | - | H | 0 | 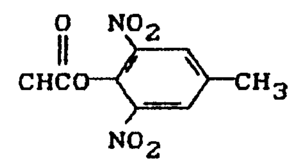 | 175 |
| 41 | CH | O | $OCH_3$ | $OCH_3$ | - | H | 0 | 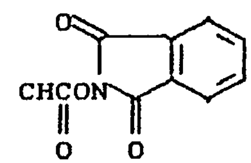 | 85 |
| 42 | CH | O | $OCH_3$ | $OCH_3$ | - | H | 0 | 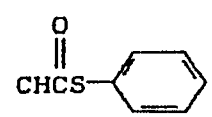 | (amorph) |
| 43 | CH | O | $OCH_3$ | $OCH_3$ | (5-)F | H | 1 | CHCN | (amorph) |

**Tabelle 1:** Beispiele für die Verbindungen der Formel (I) (Fortsetzung)

| Beisp.-Nr. | A | Q | $R^1$ | $R^2$ | $R^3$ | $R^4$ | m | Z | Schmelzpunkt (°C) |
|---|---|---|---|---|---|---|---|---|---|
| 44 | CH | O | $OCH_3$ | $OCH_3$ | - | H | 0 | $CFCOOC_2H_5$ | (amorph) |
| 45 | CH | O | $OCH_3$ | $OCH_3$ | (5-)F | H | 1 | $CFCOOC_2H_5$ | (amorph) |
| 46 | CH | O | $OCH_3$ | $OCH_3$ | - | H | 0 | 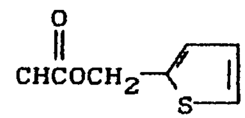 | (amorph) |
| 47 | CH | O | $OCH_3$ | $OCH_3$ | - | H | 0 | 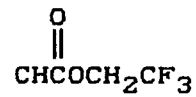 | (amorph) |
| 48 | CH | O | $OCH_3$ | $OCH_3$ | - | H | 0 | 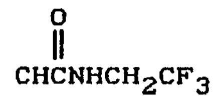 | (amorph) |
| 49 | CH | O | $OCH_3$ | $OCH_3$ | - | H | 0 | 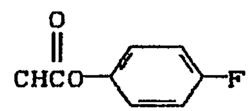 | (amorph) |
| 50 | CH | O | $OCH_3$ | $OCH_3$ | - | H | 0 | 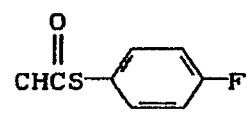 | (amorph) |
| 51 | CH | O | $OCH_3$ | $OCH_3$ | - | H | 0 | 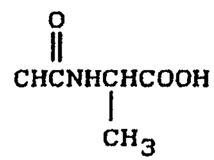 | 104 |

**Tabelle 1:** Beispiele für die Verbindungen der Formel (I) (Fortsetzung)

| Beisp.-Nr. | A | Q | $R^1$ | $R^2$ | $R^3$ | $R^4$ | m | Z | Schmelzpunkt (°C) |
|---|---|---|---|---|---|---|---|---|---|
| 52 | CH | O | $OCH_3$ | $OCH_3$ | - | H | 0 | 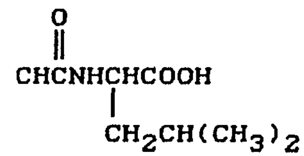 | 102 |
| 53 | CH | O | $OCH_3$ | $OCH_3$ | - | H | 0 | 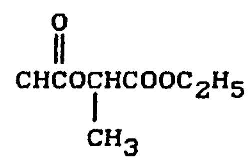 | (amorph) |
| 54 | CH | O | $OCH_3$ | $OCH_3$ | - | H | 0 | 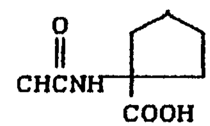 | (amorph) |
| 55 | CH | O | $OCH_3$ | $OCH_3$ | - | H | 0 | 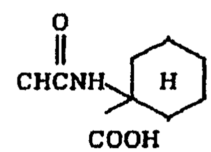 | 238 |
| 56 | CH | O | $OCH_3$ | $OCH_3$ | - | H | 0 | 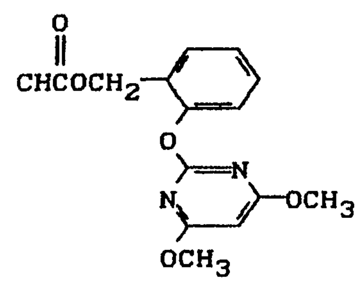 | 141 |

Tabelle 1: Beispiele für die Verbindungen der Formel (I) (Fortsetzung)

| Beisp.-Nr. | A | Q | $R^1$ | $R^2$ | $R^3$ | $R^4$ | m | Z | Schmelzpunkt (°C) |
|---|---|---|---|---|---|---|---|---|---|
| 57 | CH | O | $OCH_3$ | $OCH_3$ | - | H | 0 | 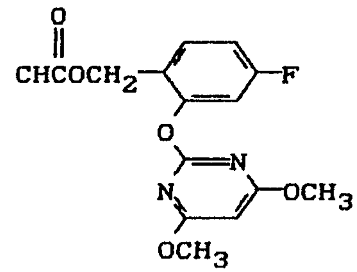 | (amorph) |
| 58 | CH | O | $OCH_3$ | $OCH_3$ | (5-)F | H | 1 | 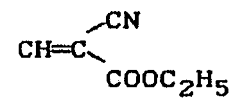 | 119 |
| 59 | CH | O | $OCH_3$ | $OCH_3$ | (5-)F | H | 1 | 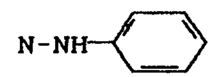 | (amorph) |
| 60 | CH | O | $OCH_3$ | $OCH_3$ | - | H | 0 | 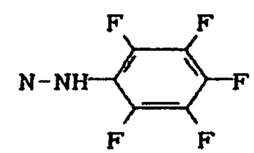 | 191 |
| 61 | CH | O | $OCH_3$ | $OCH_3$ | - | H | 0 | 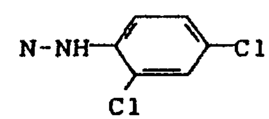 | 200 |
| 62 | CH | O | $OCH_3$ | $OCH_3$ | - | H | 0 | $N-NH-SO_2-CH_3$ | (amorph) |

Tabelle 1: Beispiele für die Verbindungen der Formel (I) (Fortsetzung)

| Beisp.-Nr. | A | Q | $R^1$ | $R^2$ | $R^3$ | $R^4$ | m | Z | Schmelzpunkt (°C) |
|---|---|---|---|---|---|---|---|---|---|
| 63 | CH | O | $OCH_3$ | $OCH_3$ | - | H | 0 | 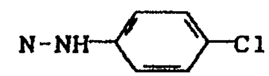 | 149 |
| 64 | CH | O | $OCH_3$ | $OCH_3$ | - | H | 0 | 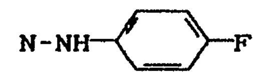 | 134 |
| 65 | CH | O | $OCH_3$ | $OCH_3$ | - | $CH_3$ | 0 | 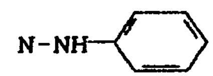 | 116 |
| 66 | CH | O | $OCH_3$ | $OCH_3$ | - | H | 0 | 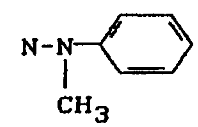 | 82 |
| 67 | CH | O | $OCH_3$ | $OCH_3$ | - | H | 0 | CFCOOH | (amorph) |
| 68 | CH | O | $OCH_3$ | $OCH_3$ | - | H | 0 | 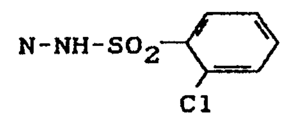 | 184 |
| 69 | CH | O | $OCH_3$ | $OCH_3$ | - | H | 0 | 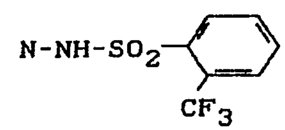 | 165 |

Tabelle 1: Beispiele für die Verbindungen der Formel (I) (Fortsetzung)

| Beisp.-Nr. | A | Q | $R^1$ | $R^2$ | $R^3$ | $R^4$ | m | Z | Schmelzpunkt ($^oC$) |
|---|---|---|---|---|---|---|---|---|---|
| 70 | CH | O | $OCH_3$ | $OCH_3$ | (5-)F | H | 1 | 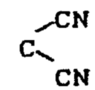 | 172 |
| 71 | C-Br | O | $OCH_3$ | $OCH_3$ | - | H | 0 | CHCOOH | 220 |
| 72 | CH | O | $OCH_3$ | $OCH_3$ | (6-)F | H | 1 | N-NH 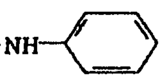 | (amorph) |
| 73 | CH | O | $OCH_3$ | $OCH_3$ | (6-)F | H | 1 | N-NH 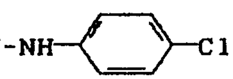 | 143 |
| 74 | CH | O | $OCH_3$ | $OCH_3$ | - | H | 0 | CHCOOH | 135 |
| 75 | CH | O | $OCH_3$ | $OCH_3$ | - | H | 0 | $CHCOOCH_3$ | 121 |
| 76 | CH | O | $OCH_3$ | $OCH_3$ | - | H | 0 | 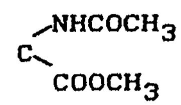 | 168 |
| 77 | CH | O | $OCH_3$ | $OCH_3$ | (6-)F | H | 1 | 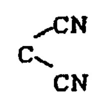 | 108 |
| 78 | CH | O | $OCH_3$ | $OCH_3$ | (6-)-F | H | 1 | CHCOOH | 163 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 79 | CH | O | $OCH_3$ | $OCH_3$ | (6-)F | H | 1 | N-NH 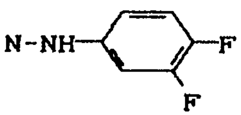 | 176 |
| 80 | CH | O | $OCH_3$ | $OCH_3$ | (6-)F | H | 1 | N-NH 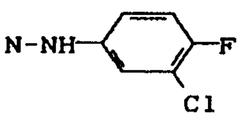 | 157 |
| 81 | CH | O | $OCH_3$ | $OCH_3$ | (6-)F | H | 1 | N-NH 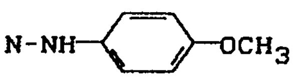 | 116 |
| 82 | CH | O | $OCH_3$ | $OCH_3$ | (6-)F | H | 1 | N-NH 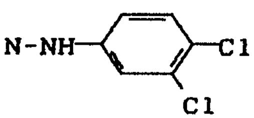 | 170 |
| 83 | CH | O | $OCH_3$ | $OCH_3$ | (5-)F | H | 1 | N-NH 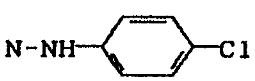 | 82 |
| 84 | CH | O | $OCH_3$ | $OCH_3$ | (5-)F | H | 1 | N-NH 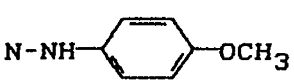 | (amorph) |
| 85 | CH | O | $OCH_3$ | $OCH_3$ | (5-)F | H | 1 | N-NH 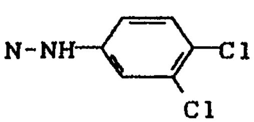 | 158 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 86 | CH | O | $OCH_3$ | $OCH_3$ | (5-)F | H | 1 | 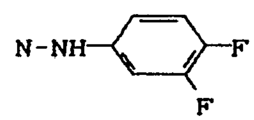 | 98 |
| 87 | CH | O | $OCH_3$ | $OCH_3$ | (5-)F | H | 1 | 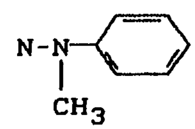 | (amorph) |
| 88 | CH | O | $OCH_3$ | $OCH_3$ | (6-)F | H | 1 | 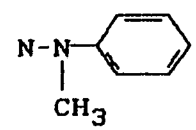 | 73 |
| 89 | CH | O | $OCH_3$ | $OCH_3$ | - | H | 0 | 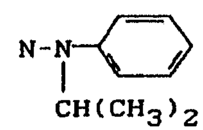 | 82 |
| 90 | N | O | $OCH_3$ | $OCH_3$ | (5-)F | H | 1 | 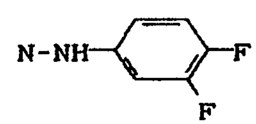 | 186 |
| 91 | N | O | $OCH_3$ | $OCH_3$ | - | H | 0 | 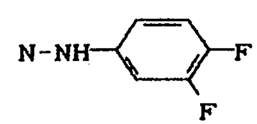 | 184 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelz-punkt (°C) |
|---|---|---|---|---|---|---|---|---|---|
| 92 | N | O | $OCH_3$ | $OCH_3$ | - | H | 0 | 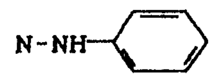 | 153 |
| 93 | N | O | $OCH_3$ | $OCH_3$ | - | H | 0 | 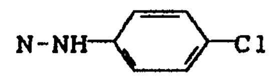 | 203 |
| 94 | CH | O | $OCH_3$ | $OCH_3$ | (5-)Cl | H | 1 | 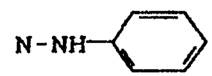 | 119 |
| 95 | CH | O | $OCH_3$ | $OCH_3$ | (5-)Cl | H | 1 | 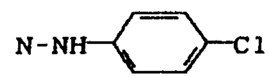 | 134 |
| 96 | N | O | $OCH_3$ | $OCH_3$ | (6-)F | H | 1 | 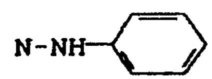 | 179 |
| 97 | N | O | $OCH_3$ | $OCH_3$ | (6-)F | H | 1 | 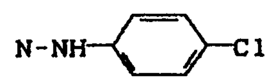 | 176 |
| 98 | CH | O | $OCH_3$ | $OCH_3$ | (5-)F | H | 1 | 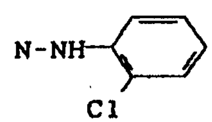 | 121 |

<u>Tabelle 1:</u> Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 99 | CH | O | $OCH_3$ | $OCH_3$ | (5-)F | H | 1 | 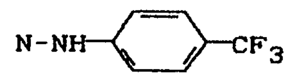 | 97 |
| 100 | CH | O | $OCH_3$ | $OCH_3$ | (5-)F | H | 1 | 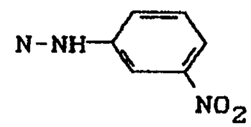 | 172 |
| 101 | CH | O | $OCH_3$ | $OCH_3$ | (5-)F | H | 1 | 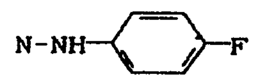 | 103 |
| 102 | CH | O | $OCH_3$ | $OCH_3$ | (5-)F | H | 1 | 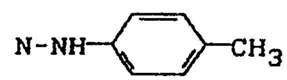 | 119 |
| 103 | CH | O | $OCH_3$ | $OCH_3$ | (5-)F | H | 1 | 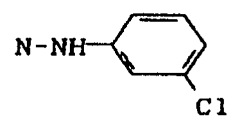 | 123 |
| 104 | CH | O | $OCH_3$ | $OCH_3$ | (5-)F | H | 1 | 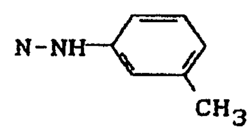 | 94 |
| 105 | CH | O | $OCH_3$ | $OCH_3$ | (6-)F | H | 1 | 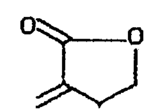 | 102 |

<u>Tabelle 1:</u> **Beispiele für die Verbindungen der Formel (I) (Fortsetzung)**

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 106 | CH | O | $OCH_3$ | $OCH_3$ | (5,6-)F | H | 2 | 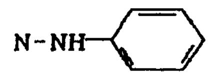 | 142 |
| 107 | CH | O | $OCH_3$ | $OCH_3$ | (5,6-)F | H | 2 | 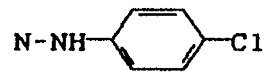 | 179 |
| 108 | CH | O | $OCH_3$ | $OCH_3$ | (5,6-)F | H | 2 | 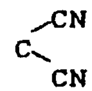 | 99 |
| 109 | CH | O | $OCH_3$ | $OCH_3$ | - | H | 0 | 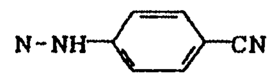 | 168 |
| 110 | N | O | $OCH_3$ | $OCH_3$ | - | H | 0 | 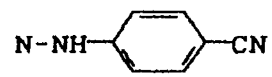 | 245 |
| 111 | CH | O | $OCH_3$ | $OCH_3$ | (5-)F | H | 1 | 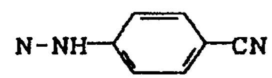 | 196 |
| 112 | CH | O | $OCH_3$ | $OCH_3$ | - | H | 0 | 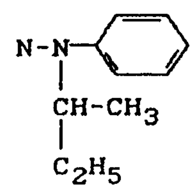 | 50 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 113 | N | O | $OCH_3$ | $OCH_3$ | - | H | 0 | 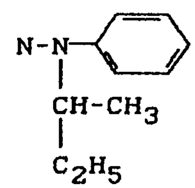 | 60 |
| 114 | CH | O | $OCH_3$ | $OCH_3$ | (5-)F | H | 1 | 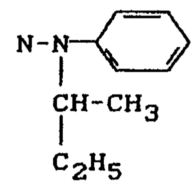 | 68 |
| 115 | CH | O | $OCH_3$ | $OCH_3$ | (6-)F | H | 1 | 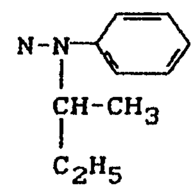 | 54 |
| 116 | CH | O | $OCH_3$ | $OCH_3$ | - | H | 0 | 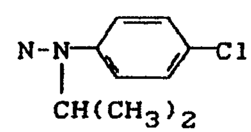 | 54 |
| 117 | N | O | $OCH_3$ | $OCH_3$ | - | H | 0 | 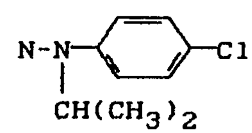 | 133 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 118 | CH | O | $OCH_3$ | $OCH_3$ | (5-)F | H | 1 | 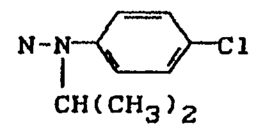 | 88 |
| 119 | CH | O | $OCH_3$ | $OCH_3$ | (6-)F | H | 1 | 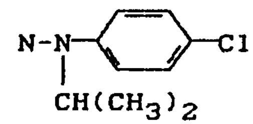 | 61 |
| 120 | CH | O | $OCH_3$ | $OCH_3$ | - | H | 0 | 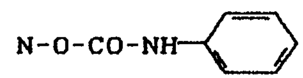 | (amorph) |
| 121 | CH | O | $OCH_3$ | $OCH_3$ | - | H | 0 | $N-NH-CO-OC(CH_3)_3$ | 105 |
| 122 | CH | O | $OCH_3$ | $OCH_3$ | - | H | 0 | 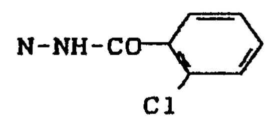 | 138 |
| 123 | CH | O | $OCH_3$ | $OCH_3$ | - | H | 0 | $N-O-CO-OCH_3$ | 86 |
| 124 | CH | O | $OCH_3$ | $OCH_3$ | - | H | 0 | $N-O-CH_2-COOC_2H_5$ | (amorph) |
| 125 | CH | O | $OCH_3$ | $OCH_3$ | - | H | 0 | 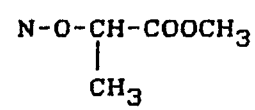 | (amorph) |

<u>Tabelle 1:</u> Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 126 | CH | O | $OCH_3$ | $OCH_3$ | (6-)$CH_3$ | H | 1 | $C(CN)_2$ | 72 |
| 127 | CH | O | $OCH_3$ | $OCH_3$ | (6-)$CH_3$ | H | 1 | N-NH 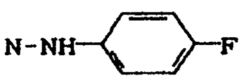 | 96 |
| 128 | CH | O | $OCH_3$ | $OCH_3$ | (6-)$CH_3$ | H | 1 | N-NH 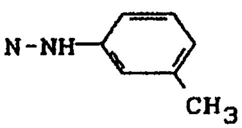 | (amorph) |
| 129 | CH | O | $OCH_3$ | $OCH_3$ | (5-)F | H | 1 | $N-NH-COOCH_3$ | 133 |
| 130 | CH | O | $OCH_3$ | $OCH_3$ | (6-)F | H | 1 | $N-NH-COOC(CH_3)_3$ | (amorph) |
| 131 | CH | O | $OCH_3$ | $OCH_3$ | (5-)Cl,(6-)F | H | 2 | N-NH 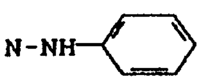 | 152 |
| 132 | CH | O | $OCH_3$ | $OCH_3$ | (5-)Cl,(6-)F | H | 2 | N-NH 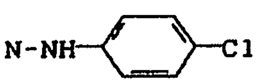 | 217 |
| 133 | CH | O | $OCH_3$ | $OCH_3$ | (5,6-)F | H | 2 | N-NH 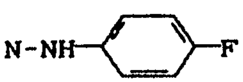 | 155 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 134 | CH | O | $OCH_3$ | $OCH_3$ | (5,6-)F | H | 2 | N-NH 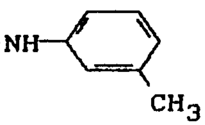 | 143 |
| 135 | CH | O | $OCH_3$ | $OCH_3$ | (6-)Cl,(5-)F | H | 2 | N-NH 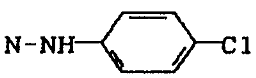 | 189 |
| 136 | CH | O | $OCH_3$ | $OCH_3$ | (5-)F | H | 1 | $N-NH-COOC(CH_3)_3$ | (amorph) |
| 137 | CH | O | $OCH_3$ | $OCH_3$ | (5,6-)F | H | 2 | N-NH 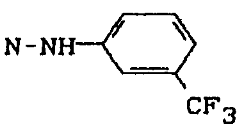 | 160 |
| 138 | CH | O | $OCH_3$ | $OCH_3$ | (5,6-)F | H | 2 | N-NH 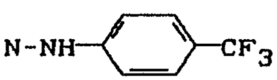 | 170 |
| 139 | CH | O | $OCH_3$ | $OCH_3$ | (5,6-)F | H | 2 | N-NH 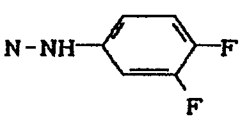 | 164 |
| 140 | CH | O | $OCH_3$ | $OCH_3$ | (5,6-)F | H | 2 | N-NH 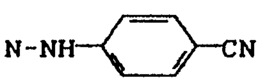 | 216 |

Tabelle 1: Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 141 | CH | O | $OCH_3$ | $OCH_3$ | (5,6-)F | H | 2 | N-NH 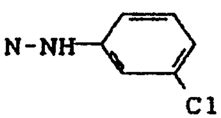 | 138 |
| 142 | CH | O | $OCH_3$ | $OCH_3$ | (5,6-)F | H | 2 | N-NH 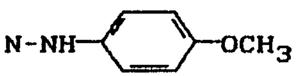 | 147 |
| 143 | CH | O | $OCH_3$ | $OCH_3$ | (5,6-)F | H | 2 | N-NH 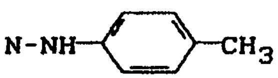 | 181 |
| 144 | CH | O | $OCH_3$ | $OCH_3$ | (5,6-)F | H | 2 | N-NH 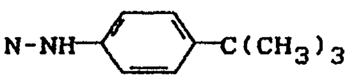 | 93 |
| 145 | CH | O | $OCH_3$ | $OCH_3$ | (5,6-)F | H | 2 | N-NH 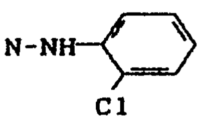 | 168 |
| 146 | CH | O | $OCH_3$ | $OCH_3$ | (5,6-)F | H | 2 | N-NH 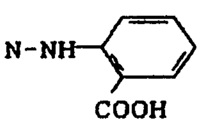 | 222 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelz-punkt (°C) |
|---|---|---|---|---|---|---|---|---|---|
| 147 | CH | O | $OCH_3$ | $OCH_3$ | (5,6-)F | H | 2 | N-NH- 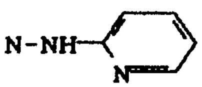 | 170 |
| 148 | CH | O | $OCH_3$ | $OCH_3$ | (5,6-)F | H | 2 | N-N- 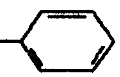<br>$CH_3$ | 82 |
| 149 | CH | O | $OCH_3$ | $OCH_3$ | (5,6-)F | H | 2 | $N-NH-SO_2CH_3$ | 140 |
| 150 | CH | O | $OCH_3$ | $OCH_3$ | (5,6-)F | H | 2 | $N-NH-SO_2$- 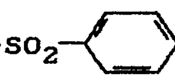 -$CH_3$ | (amorph) |
| 151 | CH | O | $OCH_3$ | $OCH_3$ | (5,6-)F | H | 2 | $N-NHCH_3$ | 75 |
| 152 | CH | O | $OCH_3$ | $OCH_3$ | (5,6-)F | H | 2 | $N-O-CH-COOCH_3$<br>$CH_3$ | (amorph) |
| 153 | CH | O | $OCH_3$ | $OCH_3$ | (5,6-)Cl | H | 2 | N-NH- 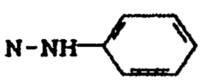 | 140 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 154 | CH | O | $OCH_3$ | $OCH_3$ | (5,6-)Cl | H | 2 | N-NH 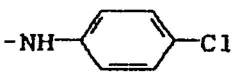 | 230 |
| 155 | CH | O | $OCH_3$ | $OCH_3$ | - | H | 0 | $N-NH_2$ | (amorph) |
| 156 | CH | O | $OCH_3$ | $OCH_3$ | (3,6-)F | H | 2 | N-NH 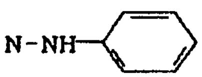 | 172 |
| 157 | CH | O | $OCH_3$ | $OCH_3$ | (3,6-)F | H | 2 | N-NH 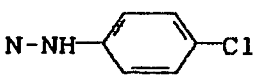 | 191 |
| 158 | CH | O | $OCH_3$ | $OCH_3$ | (6-)F | H | 1 | N-NH 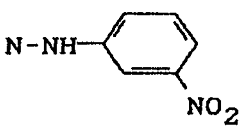 | 196 |
| 159 | CH | O | $OCH_3$ | $OCH_3$ | (5,6-)F | H | 2 | N-NH 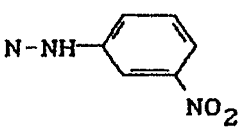 | 215 |

Tabelle 1: Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 160 | CH | O | $OCH_3$ | $OCH_3$ | (5,6-)F | H | 2 | 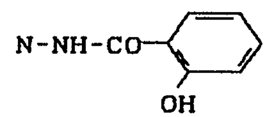 | 198 |
| 161 | CH | O | $OCH_3$ | $OCH_3$ | (5,6-)F | H | 2 | 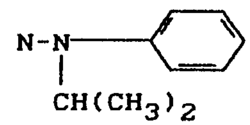 | 90 |
| 162 | CH | O | $OCH_3$ | $OCH_3$ | (5,6-)F | H | 2 | 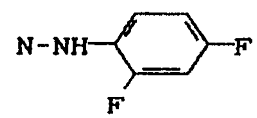 | 108 |
| 163 | CH | O | $OCH_3$ | $OCH_3$ | (5,6-)F | H | 2 | 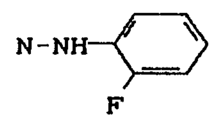 | 89 |
| 164 | CH | O | $OCH_3$ | $OCH_3$ | (5,6-)F | H | 2 | 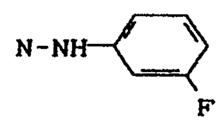 | 156 |

**Tabelle 1:** Beispiele für die Verbindungen der Formel (I) (Fortsetzung)

| Bsp.-Nr. | A | Q | $R^1$ | $R^2$ | $R^3$ | $R^4$ | m | Z | Schmelzpunkt (°C) |
|---|---|---|---|---|---|---|---|---|---|
| 165 | CH | O | $OCH_3$ | $OCH_3$ | (5,6-)F | H | 2 | N-NH 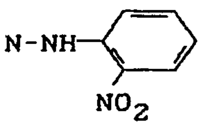 | 204 |
| 166 | CH | O | $OCH_3$ | $OCH_3$ | (5,6-)F | H | 2 | N-NH 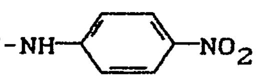 | 227 |
| 167 | CH | O | $OCH_3$ | $OCH_3$ | (5,6-)F | H | 2 | N-NH 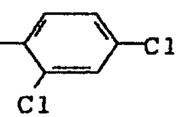 | 185 |
| 168 | CH | O | $OCH_3$ | $OCH_3$ | (5,6-)F | H | 2 | N-NH 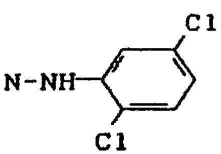 | 171 |
| 169 | CH | O | $OCH_3$ | $OCH_3$ | (5,6-)F | H | 2 | N-NH 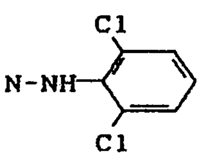 | 114 |

Die in Tabelle 1 als Beispiel 72 aufgeführte Verbindung kann beispielsweise wie folgt hergestellt werden:

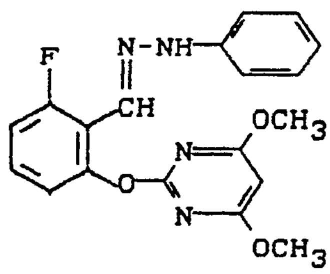

Eine Mischung aus 6-Fluor-2-(4,6-dimethoxy-pyrimidin-2-yl-oxy)-benzaldehyd (1,0 g, 3,6 mmol), Phenylhydrazin (0,39 g, 3,6 mmol) und Toluol (20 ml) wird 10 Stunden bei 20°C gerührt und anschließend eingeengt.

Man erhält 0,8 g (60% der Theorie) 6-Fluor-2-(4,6-dimethoxy-pyrimidin-2-yl-oxy)-benzaldehyd-phenylhydrazon als amorphen Rückstand.

Ausgangsstoffe der Formel (II):

Beispiel (II-1)

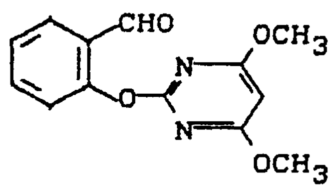

Eine Mischung aus 11,0 g (50 mMol) 4,6-Dimethoxy-2-methylsulfonyl-pyrimidin, 6,1 g (50 mMol) 2-Hydroxy-benzaldehyd, 6,9 g Kaliumcarbonat und 170 ml Acetonitril wird 5 Stunden unter Rückfluß erhitzt und anschließend eingeengt. Der Rückstand wird mit Wasser/Essigsäureethylester geschüttelt, die organische Phase abgetrennt, mit Natriumsulfat getrocknet und filtriert. Vom Filtrat wird das Lösungsmittel im Wasserstrahlvakuum sorgfältig abdestilliert.

Man erhält 7,8 g (60 % der Theorie) 2-(4,6-Dimethoxy-pyrimidin-2-yl-oxy)-benzaldehyd als festen Rückstand vom Schmelzpunkt 81 °C.

Beispiel (II-2)

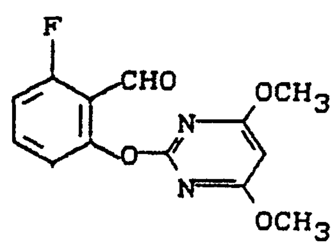

6,0 g (25 mmol) 2-(3-Fluor-phenoxy)-4,6-dimethoxy-pyrimidin werden in 150 ml Tetrahydrofuran vorgelegt, auf -78°C abgekühlt und mit 18 ml einer 15%igen Lösung von Butyllithium in Hexan versetzt. Nach einstündigem Rühren bei -78°C werden 2,2 g (30 mmol) Dimethylformamid zugetropft und das Reaktionsgemisch wird ohne weitere Außenkühlung noch eine Stunde gerührt. Dann werden 50 ml einer 5%igen wäßrigen Lösung von Natriumdihydrogenphosphat und weitere 200 ml Wasser dazugegeben und die Lösung wird dreimal mit je 150 ml Diethylether geschüttelt. Die vereinigten organischen Phasen werden mit

Natriumsulfat getrocknet und filtriert. Das Filtrat wird im Wasserstrahlvakuum eingeengt und der Rückstand durch Chromatographie (Methylenchlorid/Kieselgel) gereinigt.

Man erhält 3,9 g (56% der Theorie) 2-(4,6-Dimethoxypyrimidin-2-yl-oxy)-6-fluor-benzaldehyd vom Schmelzpunkt 93° C.

Analog Beispiel (II-2) können beispielsweise auch die folgenden neuen Verbindungen der Formel (IIa) erhalten werden:

Beispiel (II-3)

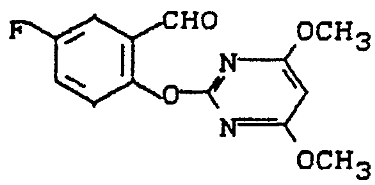

Schmelzpunkt: 114° C

Beispiel (II-4)

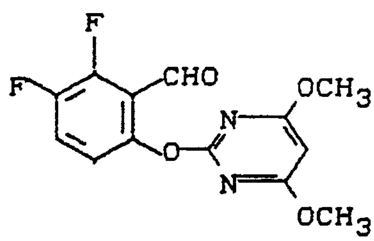

Schmelzpunkt: 113° C

Beispiel (II-5)

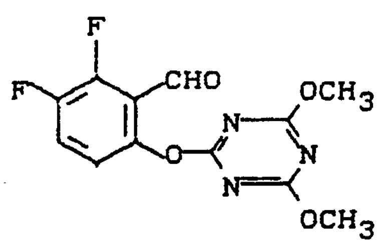

Beispiel (II-6)

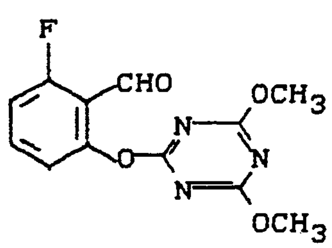

Schmelzpunkt: 94° C

Beispiel (II-7)

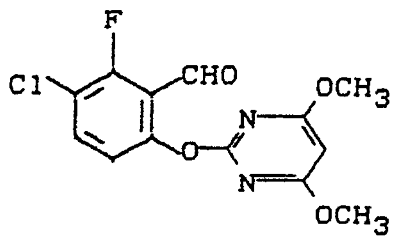

Schmelzpunkt: 125° C

Ausgangsstoffe der Formel (IV):

Beispiel (IV-1)

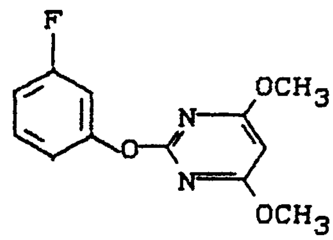

Zu 4,6-Dimethoxy-2-methylsulfonyl-pyrimidin (21,8 g, 100 mmol) in Acetonitril (300 ml) gibt man 3-Fluorphenol (11,3 g, 100 mmol) sowie Kaliumcarbonat (13,8 g). Es wird 2 Stunden unter Rückfluß zum Sieden erhitzt, in Eiswasser gegossen und mit Diethylether extrahiert. Nach dem Trocknen über Natriumsulfat wird eingeengt.

Man erhält so 21,4 g (91% der Theorie) 2-(3-Fluor-phenoxy)-4,6-dimethoxy-pyrimidin vom Schmelzpunkt 45° C.

Analog Beispiel (IV-1) können beispielsweise auch die folgenden neuen Verbindungen der Formel (IV) erhalten werden:

Beispiel (IV-2)

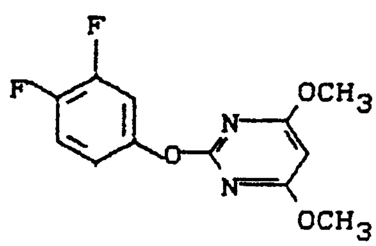

Schmelzpunkt: 85° C

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen (1), (2), (11), (23), (42), (50), (56), (57), (72), (73), (74), (76), (77), (78), (79), (80), (81), (82), (83), (84), (85), (86), (87), (88), (89), (92), (121), (122), (123), (124) und (125) starke Wirkung gegen Unkräuter.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu. und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigen beispielsweise die Verbindungen gemäß den Herstellungsbeispielen (1), (2), (7), (12), (42), (44), (50), (58), (59), (60), (61), (62), (63), (64), (65), (66), (67), (68), (69), (70), (72), (73), (74), (76), (77), (78), (79), (80), (81), (82), (83), (84), (85), (86), (87), (88), (89), (90), (91), (92), (93), (120), (121), (123), (124) und (125) starke Wirkung gegen Unkräuter.

## Patentansprüche

1. Substituierte Azine der allgemeinen Formel (I)

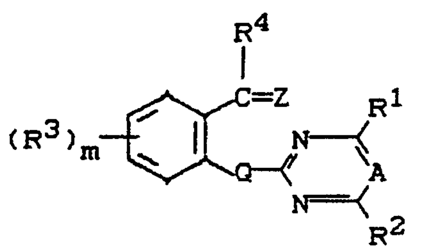

(I)

in welcher

m für die Zahlen 0, 1, 2 oder 3 steht,
A für Stickstoff oder eine C-X-Gruppierung steht, wobei X für Wasserstoff oder Halogen steht,
Q für Sauerstoff oder Schwefel steht,

$R^1$ und $R^2$ gleich oder verschieden sind und für Wasserstoff, Halogen, Alkyl, Halogenalkyl, Alkoxyalkyl, Alkoxy, Halogenalkoxy, Alkylthio, Alkylamino oder Dialkylamino stehen,

$R^3$ für Amino, Hydroxy, Halogen, Alkyl, Halogenalkyl, Alkoxy, Halogenalkoxy, Alkylamino, Dialkylamino, Alkylcarbonylamino, Alkoxycarbonylamino oder Alkylsulfonylamino steht,

$R^4$ für Wasserstoff oder Alkyl steht und

Z für eine der nachstehenden Gruppierungen steht:

$N-R^5$ oder

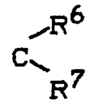 ,

wobei

$R^5$ für Wasserstoff, Amino oder für jeweils gegebenenfalls substituiertes Alkyl, Alkenyl, Alkinyl, Aralkyl, Aryl, Alkoxycarbonyloxy, Arylaminocarbonyloxy, Carboxyalkoxy, Alkoxycarbonylalkoxy, Alkylamino, Dialkylamino, Aralkylamino, Arylamino, N-Alkyl-N-arylamino, Alkylcarbonylamino, Arylcarbonylamino, Heteroarylamino, Heteroarylcarbonylamino, Alkoxycarbonylamino, Alkylsulfonylamino oder Arylsulfonylamino steht,

$R^6$ für Wasserstoff, Halogen, Cyano, Carboxy, Alkoxycarbonyl, Alkylcarbonylamino oder Dialkoxyphosphoryl steht und

$R^7$ für Formyl, Cyano, Carboxy, Hydroxymethyl, Carbamoyl oder für jeweils gegebenenfalls substituiertes Alkoxycarbonyl, Cycloalkyloxycarbonyl, Aralkoxycarbonyl, Aryloxycarbonyl, Heterocyclylalkoxycarbonyl, Alkylthiocarbonyl, Aralkylthiocarbonyl, Arylthiocarbonyl, Alkylaminocarbonyl, Cycloalkylaminocarbonyl, Aralkylaminocarbonyl, Arylaminocarbonyl, Dialkylaminocarbonyl, Alkylhydrazinocarbonyl, Arylhydrazinocarbonyl, Pyrrolidinylcarbonyl, Piperidinylcarbonyl, Morpholinylcarbonyl, Piperazinylcarbonyl, Phthalimidoxycarbonyl, für die Gruppierung

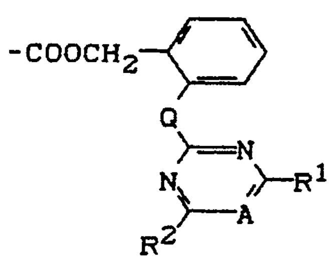

in welcher A, Q, $R^1$ und $R^2$ die oben angegebenen Bedeutungen haben, steht oder zusammen mit $R^6$ für die Gruppierung $-CO-O-(CH_2)_n-$ steht, wobei n für die Zahlen 1 bis 4 steht,

**2.** Substituierte Azine der allgemeinen Formel (I) gemäß Anspruch 1, dadurch gekennzeichnet, daß darin

m für die Zahlen 0, 1 oder 2 steht,

A für Stickstoff oder eine C-X-Gruppierung steht, wobei X für Wasserstoff oder Halogen steht,

Q für Sauerstoff oder Schwefel steht,

$R^1$ und $R^2$ gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen,

$R^3$ für Amino, Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, Di-($C_1$-$C_2$-alkyl)-amino, $C_1$-$C_4$-Alkyl-carbonylamino, $C_1$-$C_4$-Alkoxy-carbonylamino oder $C_1$-$C_4$-Alkylsulfonylamino steht,

$R^4$ für Wasserstoff oder $C_1$-$C_4$-Alkyl steht und

Z für eine der nachstehenden Gruppierungen steht:

$N-R^5$ oder

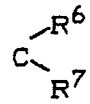 ,

wobei

$R^5$ für Wasserstoff, Amino oder für jeweils gegebenenfalls durch Halogen substituiertes $C_1$-$C_6$-Alkyl, $C_3$-$C_6$-Alkenyl, $C_3$-$C_6$-Alkinyl, Carboxy-$C_1$-$C_2$-alkoxy, $C_1$-$C_6$-Alkoxycarbonyloxy, $C_1$-$C_4$-Alkoxycarbonyl-$C_1$-$C_2$-alkoxy, $C_1$-$C_6$-Alkylamino, Di-($C_1$-$C_2$-alkyl)-amino, $C_1$-$C_6$-Alkylcarbonylamino, $C_1$-$C_6$-Alkoxy-carbonylamino, $C_1$-$C_6$-Alkyl-sulfonylamino, oder für jeweils gegebenenfalls durch Nitro, Hydroxy, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenylaminocarbonyloxy, Phenylamino, Phenyl-$C_1$-$C_4$-alkylamino, N-($C_1$-$C_4$-alkyl)-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Halogen, Cyano, Carboxy, $C_1$-$C_6$-Alkoxy-carbonyl, $C_1$-$C_6$-Alkyl-carbonylamino oder Di-($C_1$-$C_4$-alkoxy)-phosphoryl steht und

$R^7$ für Formyl, Cyano, Carboxy, Hydroxymethyl, Carbamoyl oder für jeweils gegebenenfalls durch Halogen, Carboxy oder $C_1$-$C_4$-Alkoxy-carbonyl substituiertes $C_1$-$C_6$-Alkoxy-carbonyl, $C_5$-$C_6$-Cycloalkyloxy-carbonyl, $C_1$-$C_6$-Alkylthio-carbonyl, $C_1$-$C_6$-Alkylamino-carbonyl oder $C_5$-$C_6$-Cycloalkylamino-carbonyl, für Di-($C_1$-$C_2$-alkyl)-amino-carbonyl, für $C_1$-$C_4$-Alkyl-amino-carbonyl-$C_1$-$C_4$-alkoxy-carbonyl, für Di-($C_1$-$C_2$-alkyl)-amino-carbonyl-$C_1$-$C_4$-alkoxy-carbonyl, für Phenylaminocarbonyl-$C_1$-$C_4$-alkoxy-carbonyl, für N-Methyl-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthiocarbonyl, Phenylaminocarbonyl, Phenyl-$C_1$-$C_4$-alkylamino-carbonyl, N-($C_1$-$C_4$-Alkyl)-phenylamino-carbonyl oder Phenylhydrazinocarbonyl, für Phthalimidoxycarbonyl, für die Gruppierung

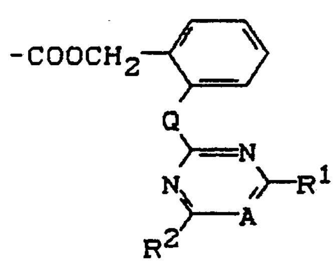

in welcher A, Q, $R^1$ und $R^2$ die oben als bevorzugt angegebenen Bedeutungen haben, steht oder zusammen mit $R^6$ für die Gruppierung $-CO-O-(CH_2)_n-$ steht, wobei n für die Zahlen 2 oder 3 steht.

3. Substituierte Azine der allgemeinen Formel (I) gemäß Anspruch 1, dadurch gekennzeichnet, daß darin

m für die Zahlen 0, 1 oder 2 steht,

A für Stickstoff oder eine CH-Gruppierung steht,

Q für Sauerstoff steht,

$R^1$ für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino steht,

$R^2$ für Wasserstoff, Chlor, Methyl, Ethyl, Trifluormethyl, Methoxy, Ethoxy, Difluormethoxy, Methylthio, Methylamino, Ethylamino oder Dimethylamino steht,

$R^3$ für Amino, Hydroxy, Fluor, Chlor, Brom, Methyl, Ethyl, Trifluormethyl, Methoxy, Difluormethoxy, Trifluormethoxy, Methylamino, Dimethylamino, Acetylamino, Methoxycarbonylamino oder Methylsulfonylamino steht,

$R^4$ für Wasserstoff oder Methyl steht und

Z für eine der nachstehenden Gruppierungen steht:

N-$R^5$ oder

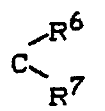 ,

wobei

$R^5$ für Wasserstoff, Amino, für Methyl, Ethyl, Propyl, Isopropyl, Butyl, Isobutyl, sec-Butyl, tert-Butyl, Allyl, Propargyl, Carboxymethoxy, Carboxyethoxy, Methoxycarbonyloxy, Ethoxycarbonyloxy, Propoxycarbonyloxy, Butoxycarbonyloxy, Methoxycarbonylmethoxy, Ethoxycarbonylmethoxy, Methoxycarbonylethoxy, Ethoxycarbonylethoxy, Methylamino, Ethylamino, Propylamino, Isopropylamino, Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Hydroxy, Cyano, Fluor, Chlor, Brom, Methyl, Trifluormethyl, Methoxy, Trifluormethoxy, Methylthio oder Trifluormethylthio substituiertes Phenyl, Benzyl, Phenylaminocarbonyloxy, Phenylamino, Benzylamino, N-Methylphenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Fluor, Chlor, Cyano, Carboxy, $C_1$-$C_4$-Alkoxy-carbonyl, $C_1$-$C_4$-Alkyl-carbonylamino, Dimethoxyphosphoryl oder Diethoxyphosphoryl steht und

$R^7$ für Formyl, Cyano, Carboxy, Hydroxymethyl, Carbamoyl, für jeweils gegebenenfalls durch Fluor, Chlor, Carboxy oder $C_1$-$C_4$-Alkoxy-carbonyl substituiertes $C_1$-$C_4$-Alkoxy-carbonyl, $C_5$-$C_6$-Cycloalkyloxy-carbonyl, $C_1$-$C_4$-Alkylthiocarbonyl, $C_1$-$C_4$-Alkylamino-carbonyl oder $C_5$-$C_6$-Cycloalkylamino-carbonyl, für Dimethylaminocarbonyl, für $C_1$-$C_4$-Alkylaminocarbonyl-$C_1$-$C_4$-alkoxy-carbonyl, für Dimethylaminocarbonyl-$C_1$-$C_4$-alkoxy-carbonyl, für N-Methylphenylaminocarbonyl-$C_1$-$C_4$-alkoxy-carbonyl, für jeweils gegebenenfalls durch Methyl und/oder Ethyl substituiertes Pyrrolidinyl-carbonyl, Piperidinyl-carbonyl, Morpholinyl-carbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-phenylaminocarbonyl, Phenylhydrazinocarbonyl, für Phthalimidoxycarbonyl, für die Gruppierung

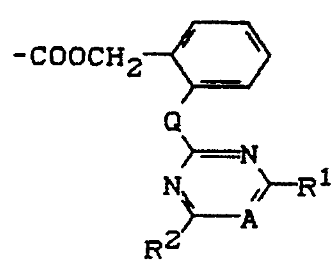

in welcher A, Q, $R^1$ und $R^2$ die oben angegebenen Bedeutungen haben, steht oder zusammen mit $R^6$ für die Gruppierung -CO-O-$CH_2CH_2$- steht.

4. Substituierte Azine der allgemeinen Formel (I) gemäß Anspruch 1, dadurch gekennzeichnet, daß darin
   m für die Zahlen 0, 1 oder 2 steht,
   A für eine CH-Gruppierung steht,
   Q für Sauerstoff steht,
   $R^1$ für Methoxy steht,
   $R^2$ für Methoxy steht,
   $R^3$ für Fluor, Chlor oder Methyl steht,
   $R^4$ für Wasserstoff steht und
   Z die in Anspruch 3 angegebene Bedeutung hat.

5. Verfahren zur Herstellung von substituierten Azinen der allgemeinen Formel (I) gemäß Anspruch 1, dadurch gekennzeichnet, daß man entsprechende Carbonylverbindungen der allgemeinen Formel (I)

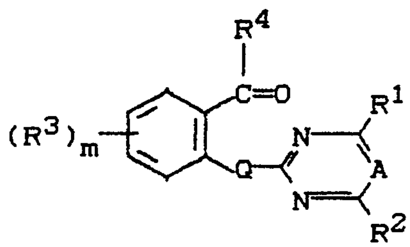

(II)

in welcher

m, A, Q, $R^1$, $R^2$, $R^3$ und $R^4$ die in Anspruch 1 angegebene Bedeutung haben,

mit Amino- oder Methylen-Verbindungen der allgemeinen Formel (III)

$H_2Z$ (III)

in welcher
   Z die in Anspruch 1 angegebene Bedeutung hat,
gegebenenfalls in Gegenwart eines Reaktionshilfsmittels und gegebenenfalls in Gegenwart eines Verdünnungsmittels umsetzt und gegebenenfalls die hierbei erhaltenen Produkte anschließend nach üblichen Methoden in andere Derivate gemäß der Definition der Verbindungen der Formel (I) umwandelt.

6. Herbizide Mittel, gekennzeichnet durch einen Gehalt an mindestens einem substituierten Azin der allgemeinen Formel (I) gemäß Anspruch 1.

7. Verwendung von substituierten Azinen der Formel (I) gemäß Anspruch 1 zur Bekämpfung von unerwünschtem Pflanzenwachstum.

8. Verfahren zur Herstellung von herbiziden Mitteln, dadurch gekennzeichnet, daß man substituierte Azine der Formel (I) gemäß Anspruch 1 mit Streckmitteln und/oder oberflächenaktiven Mitteln vermischt.

9. Carbonylverbindungen der Formel (IIa)

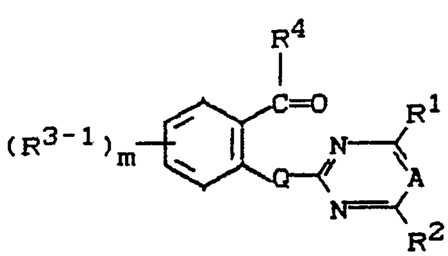

(IIa)

in welcher

| | |
|---|---|
| A | für Stickstoff oder eine C-X-Gruppierung steht, wobei X für Wasserstoff oder Halogen steht, |
| Q | für Sauerstoff oder Schwefel steht, |
| $R^1$ und $R^2$ | gleich oder verschieden sind und für Wasserstoff, Halogen, Alkyl, Halogenalkyl, Alkoxyalkyl, Alkoxy, Halogenalkoxy, Alkylthio, Alkylamino oder Dialkylamino stehen, |
| $R^4$ | für Wasserstoff oder Alkyl steht, |
| m | für die Zahlen 1 oder 2 steht und |
| $R^{3-1}$ | für Fluor und gegebenenfalls zusätzlich für Chlor steht. |

**10.** Halogenverbindungen der Formel (IV)

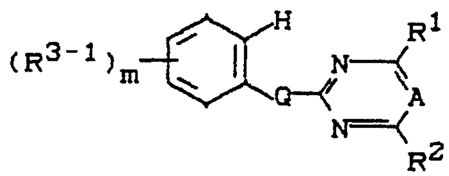

(IV)

in welcher

| | |
|---|---|
| A | für Stickstoff oder eine C-X-Gruppierung steht, wobei X für Wasserstoff oder Halogen steht, |
| Q | für Sauerstoff oder Schwefel steht, |
| $R^1$ und $R^2$ | gleich oder verschieden sind und für Wasserstoff, Halogen, Alkyl, Halogenalkyl, Alkoxyalkyl, Alkoxy, Halogenalkoxy, Alkylthio, Alkylamino oder Dialkylamino stehen, |
| m | für die Zahlen 1 oder 2 steht und |
| $R^{3-1}$ | für Fluor und gegebenenfalls zusätzlich für Chlor steht. |